# EUROPEAN PATENT APPLICATION

(11) **EP 3 449 812 A1**
(43) Date of publication of application: **06.03.2019**
(21) Application number: 17460057.7
(22) Date of filing: 05.09.2017
(51) Int. Cl.: A61B 3/16, A61B 3/10, A61B 3/107

(54) **OPHTHALMIC ANALYSIS SYSTEM FOR MEASURING THE INTRAOCULAR PRESSURE IN THE EYE**

(71) Applicant: Frey Spolka Jawna, 05-502 Piaseczno (PL)
(72) Inventor: Frey, Wojciech, 02-972 Warsaw (PL); Frey, Jacek, 05-502 Piaseczno (PL)

(57) **Abstract**

Ophthalmic analysis system for measuring cornea thickness and elasticity for precise intraocular pressure in the eye calculation is presented. The device according to the invention allows for simple measurement of corneal thickness and elasticity using low frame rate camera and short pulses of slit light to capture images of cornea deformation by applanation puff.

## Description

### FIELD OF THE INVENTION

The invention relates to an ophthalmic analysis system for noncontact measurement of intraocular pressure in the eye.

### BACKGROUND OF THE INVENTION

Increased intraocular pressure in the eye can trigger serious health impairments, in particular the optic nerve can be damaged and result in restrictions of the visual field.

There are three basic methods for intraocular pressure measurement, impression tonometry, applanation tonometry and noncontact tonometry. The first two methods are contact type methods, meaning elements of a measuring device must contact and apply a certain force directly onto the cornea of the eye in order to obtain a value for the eye's intraocular pressure.

Noncontact applanation tonometers, also called air-puff tonometers, developed by Bernard Grolman and set forth in U.S. Patent No. 3,585,849, are known for noncontact measurement of the eye's intraocular pressure. In this type of tonometers, a puff of air is blown onto the cornea of the subject eye from a nozzle and applanation of the cornea is optically detected. Since the invention of the air puff tonometer many techniques have been developed and implemented to increase the accuracy of intraocular pressure measurements.

Certain cornea parameters like thickness and elasticity, which can be described as the cornea's susceptibility to applied force, have significant impact on precise measurement of the eye's intraocular pressure. Most of the currently available air puff type tonometers have embedded calculation formulas for correction of the intraocular pressure by considering the cornea thickness values that were measured with an external pachymeter. Some of the devices embed a pachymeter and an air puff type tonometer in one device, an example of which is described in European Patent EP 2095761A1.

European patent EP 2 397 068 A1 describes a new method of intraocular pressure calculation based on analysis of images of cornea deformation. Patent US 2012/0065488 A1 describes an ophthalmic analysis system for intraocular pressure measurement based on such image analysis. This method uses parameters like cornea thickness and cornea elasticity. A disadvantage of the device described in patent application US 2012/0065488 A1 is the large amount of data captured during the measurement cycle and as a result the high computing power requirement of the device for calculation of the eye's intraocular pressure.

The objective of the present invention is to provide a compact and simple in construction device for measuring intraocular pressure capable of measuring cornea thickness and cornea elasticity as well as providing appropriate correction values based on cornea thickness and elasticity in order to provide an accurate measurement of intraocular pressure.

Accordingly, a primary object of the present invention is to provide a non contact air puff type tonometer that uses only the images of cornea deformation that are important for cornea flexibility calculation. This is achieved by on-the-fly analysis of air pressure and the applanation signals as well as the triggering of a cornea image capture sequence during defined moments of the applanation signal. Moreover to allow the use of slow speed cameras and capture an image free of movement distortions, the frame is created by using flash pulses of light. The intraocular pressure in the eye is then calculated based on data recorded from the air puff pressure signal and the applanation signal which has been corrected for the corneal thickness and corneal elasticity parameters calculated from the corneal deformation images captured during the measurement cycle.

In order to achieve the object mentioned above, the present invention provides for a system for air puff generation, an optical system for projecting a slit onto the subject cornea which creates an observation of the cross-sectional plane of the eye, a camera system for recording cross-sectional images of the cornea and an electronic control module for recording and on-the-fly analysis of air puff pressure and applanation signals and for controlling slit light intensity as well as managing the cross-sectional cornea image capture sequence.

### BRIEF DESCRIPTION OF DRAWINGS

The features of the invention are shown in the drawings as an example hereinafter.

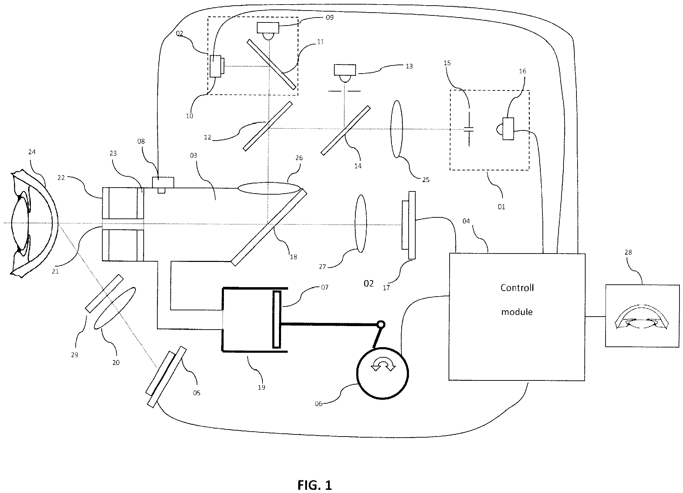

FIG. 1: presents the structure of one of the embodiments of the analysis system according to the invention for measuring intraocular pressure; Slit projection module 01 together with lenses 25 and 26 is used to project a slit image onto the cornea; Camera module 17 together with lens 27 is used as an alignment camera; Actuator 06 together with piston 07, piston chamber 19, compression chamber 03 and nozzle orifice is used to generate an air puff for cornea applanation; camera sensor 05, lens 20 and filter 29 capture cross-sectional images of the subject cornea; applanation detection module 02 is used to transmit light towards the subject cornea and detects light reflected from said cornea during cornea deformation by air-puff; Control module 04 is used to synchronize operation of all modules of the device and to calculate intraocular pressure and display measured values to the operator.

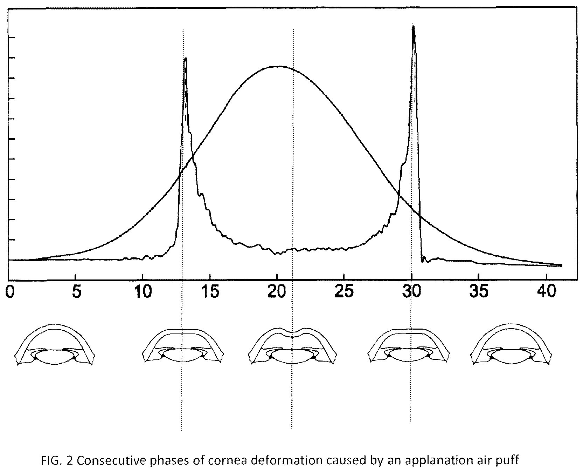

FIG. 2: Presents the correlation between air pressure in the compression chamber 03, applanation curve detected by applanation module 02 and graphical representation of the corresponding cornea deformation phases.

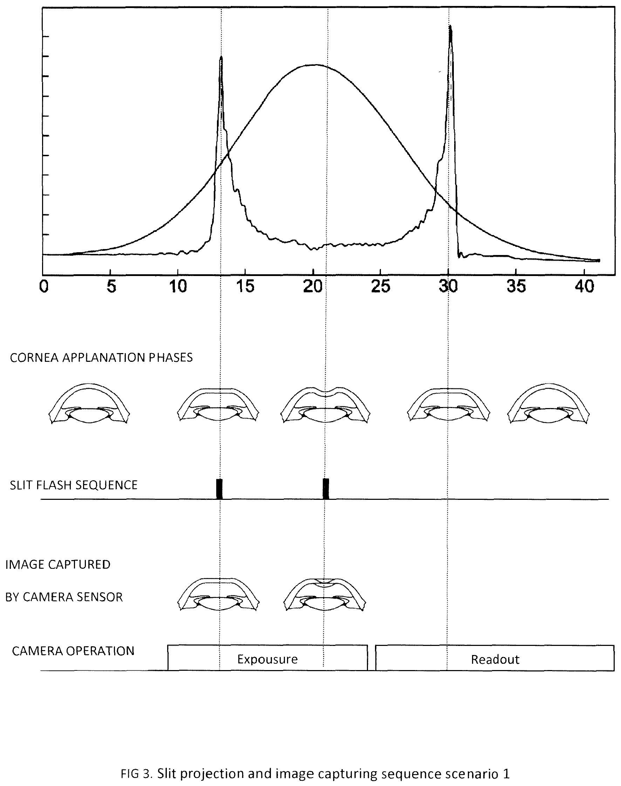

FIG. 3:Presents a slit projection and image capturing sequence by camera 05 in relation to a cornea applanation sequence in case of use of a slow image sensor, snapshot images are overlaid on each other. During Exposure time period camera sensor 05 is capturing image information only during flash pulses of slit light, Readout is a time period when the sensor 05 is transmitting captured image to control circuit 04.

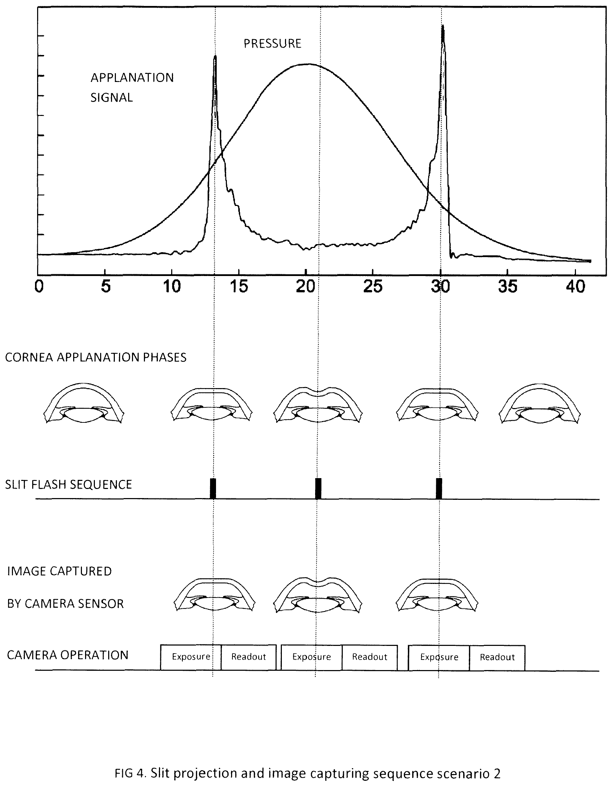

FIG. 4: Presents a slit projection and image capturing sequence by camera 05 in relation to a cornea applanation sequence in case of use of an image sensor capable of reading the entire image area between consecutive slit light flashes.

### DETAILED DESCRIPTION

The analysis system presented schematically in FIG. 1 is used to measure intraocular pressure of the eye 24 and apply correction parameters related to corneal thickness and corneal elasticity.

In order to measure corneal thickness the slit of light, generated in slit projector unit 01 comprising of light source 16 and the slit 15, is projected by the optical system comprising of lens 25 and lens 26 onto the cornea. The slit projected onto the subject cornea creates a cross sectional image which is recorded by the camera system comprising of camera sensor 05 and imaging lens 20 with optional optical filter 29. To minimize influence of ambient light and assure high contrast of cross-sectional images, filter 29 passes through only light of wavelength close to the one emitted by slit light source 16. A captured cross-sectional image is stored, analyzed and the corneal thickness is calculated from the image by control circuit 04. Corneal thickness value, will be used to apply a correction coefficient to intraocular pressure value calculated from analysis of application signal.

To measure intraocular pressure of the eye 24 the cornea must be deformed. An actuating device comprising of piston chamber 19 and piston 07 driven by electrically controlled actuator 06 is used to generate high pressure in closed compression chamber 03. Piston 07 can be moved rapidly by actuator 06 within piston chamber 19 and produce increased pressure in compression chamber 03. Compression chamber 03 has a nozzle orifice 21 pointing in the direction of a subject eye 24. By expelling high pressure air from air compression chamber 03 through nozzle orifice 21 an air pulse is generated and directed towards the cornea of the subject eye. For monitoring air pressure in compression chamber 03 pressure detector 08 is used. Signal from pressure detector 08 is recorded and analyzed by control module 04. The air pulse interacting with the subject cornea will create the cornea's deformation.

Applanation signal detector module 02 comprising of light source 09, light detector 10 and beam splitter plate 11 is used to track consecutive phases of cornea deformation. The light signal from source 09 is projected through beam splitter 12 and lens 26 and is directed by beam splitter 18 onto the subject cornea. A certain portion of the light is reflected back from the cornea and returns to applanation signal module 02 at which it is measured by light detector 10. The signal from light detector 10 is proportional to the area of the cornea applanated by an air puff and is recorded and analyzed by control module 04.

For capturing cross sectional image of cornea deformation, operation of camera 05 is synchronized by control module 04 with slit light emission from slit projector 01. Control module 04 analyzes the signal from applanation signal detector 02 and upon detection of a predefined state can switch on slit generator 01.

Consecutive phases of cornea deformation and its relation to an air pressure value in compression chamber 03 and an applanation signal value from light detector 10 are presented in FIG. 2.

To detect cornea behavior under an applied air puff impulse, consecutive cross sectional images of the slit projected by slit generator 01 onto the subject cornea 24 are recorded by camera 05. Images are stored for analysis and cornea elasticity parameter calculation by control circuit 04. To allow use of a low frame rate camera and avoid motion artifacts of cornea movement during cornea deformation, control module 04 is used to produce short flash impulses of slit light by slit generation module 01. If the camera 05 is fast enough to transfer captured data to the control module 04 between consecutive slit flashes, images of cornea deformation will be stored as separate data and later used for analysis and calculations, this scenario is illustrated on Fig 4. In case camera 05 is not fast enough to transfer images of consecutive phases of cornea deformation between slit flash pulses, then the images will overlap on each other as presented in Fig. 3. In this scenario captured data can be transferred to control module 04 for analysis and cornea elasticity calculation. Overlaid cornea deformation images can be separated by software processing. Additionally to simplify separation of overlapped images slit generation module 01 can use more then one color for slit generation and color sensitive camera 05 can be used.

Cross-sectional images of cornea deformation stored by control module 04 are software processes to calculate mechanical properties of the cornea and to calculate coefficients used to correct value of intraocular pressure measured by the device.

## Claims

1. A non-contact tonometer comprising of:
a piston;
a drive unit which is connected to the piston, to drive the piston;
a fluid injection unit to direct air, pressurized by the piston, onto subject cornea;
an intraocular pressure measuring unit, projecting light onto subject cornea and using light reflected from cornea, to optically detect deformation of the cornea caused by air puff;
a slit projection unit, which projects slit image onto subject cornea, capable of producing short pulses of slit light;
a camera for recording cross-sectional images of subject cornea, where the camera is aligned with the slit image projected onto the cornea according to Schempflug principle;
an electronic control module synchronizing operation of non-contact tonometer modules capable of on the fly analyzing cornea deformation signal and triggering slit projection and cross sectional image capturing sequence.

2. A non-contact tonometer according to claim 1, capable of measuring thickness of the cornea using cross sectional image of the cornea;
where cornea thickness value is used to correct intraocular pressure value measured by the device.

3. A non-contact tonometer according to claim 1, where control module can be programmed to trigger slit flash at specific time location defined by applanation signal curve and capture such created cross-sectional image of deformed cornea;
where control module can produce one or multiple slit flash and capture sequence during single intraocular pressure measurement cycle.

4. A non-contact tonometer according to claim 3, where control module can produce multiple slit flashes during single cross-sectional camera frame exposure to produce overlaying cross-sectional images of cornea deformation.

5. A non-contact tonometer according to claim 3, where control module can produce multiple sequences of slit flash and cross-sectional image capture during single intraocular pressure measurement cycle.

6. A non-contact tonometer according to claim 4, using color sensitive camera for cross-sectional image capturing and slit projection unit using multiple color light source.
